# EUROPEAN PATENT APPLICATION

(11) **EP 4 006 134 A1**
(43) Date of publication of application: **01.06.2022**
(21) Application number: 20843314.4
(22) Date of filing: 21.07.2020
(51) Int. Cl.: C12M 1/00, C12M 1/18, C12M 3/00, C12N 5/079, C12N 5/0793

(54) **CULTURE METHOD AND CULTURE VESSEL**

(30) Priority: 22.07.2019 JP 2019134262; 28.02.2020 JP 2020034347
(71) Applicant: Jiksak Bioengineering, Inc., Kawasaki-shi, Kanagawa, 212-0032 (JP)
(72) Inventor: KAWADA Jiro, Kawasaki-shi, Kanagawa 212-0032 (JP); KASAI Akira, Tokyo 100-8246 (JP)
(74) Representative: Scott, Stephen John
(86) International application number: PCT/JP2020/028338
(87) International publication number: WO 2021/015213

(57) **Abstract**

To culture cells in each of a plurality of wells in a culture vessel under optimal culture conditions depending on the type of the cells cultured in the well, a culture method comprises: filling a first well 1 and a second well 2 in a culture vessel with a culture medium for neurons, the first well 1 and the second well 2 communicating with each other through a flow channel 3; seeding the first well 1 with neurons N; culturing axons a of the neurons N until the axons a of the neurons N reach the second well 2 through the flow channel 3 and also block the flow channel 3; and thereafter removing the culture medium in the second well 2, filling the second well 2 with a culture medium for cells to be co-cultured with the neurons N, seeding cells C to be co-cultured in the second well 2, and culturing the cells C together with the axons a of the neurons N.

## Description

### TECHNICAL FIELD

The present disclosure relates to a culture method and a culture vessel for cells, and particularly relates to a culture method for neurons (nerve cells), a co-culture method for neurons and cells other than neurons, and a culture vessel for culturing them.

### BACKGROUND

In living organisms, axons extending from neurons are joined to cells other than neurons, such as skeletal muscle cells or skin cells. Co-culture of neurons and other cells is therefore expected to serve as an effective screening tool in the field of drug discovery. For example, WO 2017/187696 A1 (PTL 1) discloses culturing a cell mass of neurons in a predetermined culture vessel, extending the axons of the neurons in a bundle, and joining the axon terminals to a skeletal muscle.

In the search phase of drug discovery, high-throughput drug screening is needed. As a culture vessel used for high-throughput screening, a multiwell plate with at least 96 wells is known. For example, JP 2018-536424 A (PTL 2) discloses creating a neuron network by connecting wells of a multiwell plate and co-culturing neurons and cells other than neurons.

### CITATION LIST

### Patent Literature

PTL 1: WO 2017/187696 A1
PTL 2: JP 2018-536424 A

### SUMMARY

### (Technical Problem)

Co-culture involves seeding different cells in a culture space of a culture vessel and culturing them. Since a suitable culture medium differs depending on the cell type, it is difficult to culture different cells in the same vessel. Although this might be overcome by developing a culture medium capable of culturing both cells, developing such a culture medium is not easy.

For co-culture of neurons and cells other than neurons, it is considered necessary to use respective different culture mediums suitable for culture in a well for culturing the cell bodies of neurons and in a well for culturing the axons extending from the cell bodies and cells such as skeletal muscle cells to be joined to the axons. In the case of not performing co-culture, too, it is considered necessary to use respective different culture mediums suitable for culture in a well for culturing the cell bodies of neurons and in a well for culturing the tips of the axons extending from the cell bodies. Neither PTL 1 nor PTL 2 recites a specific means for using different culture mediums between wells that communicate with each other.

It could therefore be helpful to provide a culture method and a culture vessel that can culture cells in each of a plurality of wells in the culture vessel under optimal culture conditions depending on the type of the cells cultured in the well.

### (Solution to Problem)

In view of the foregoing technological background, we conducted intensive studies, and consequently discovered that wells communicating with each other through a flow channel can be separated from each other by controlling the number of neurons and the size (cross-sectional area) of the flow channel and blocking the flow channel with axons extending in the flow channel.

We further discovered that, by limiting the size (cross-sectional area) and length of the flow channel connecting the wells in the culture vessel to specific ranges, liquids such as culture mediums filling the respective wells connected by the flow channel can be held without mixing with each other, even in the case where cells are not present in the flow channel.

The present disclosure is based on these discoveries, and is intended to advantageously solve the problem stated above. A first aspect of the present disclosure is a culture method of co-culturing a plurality of types of cells including neurons in a culture vessel having a plurality of wells, the culture method comprising: filling a first well and a second well included in the plurality of wells in the culture vessel with a culture medium for the neurons, and seeding the neurons in the first well, the first well and the second well communicating with each other through a flow channel; culturing axons of the neurons until the axons of the neurons reach the second well through the flow channel and also block the flow channel; and thereafter removing the culture medium in the second well, filling the second well with a culture medium for co-cultured cells to be co-cultured with the neurons, seeding the co-cultured cells in the second well, and culturing the co-cultured cells together with the axons of the neurons.

By culturing the axons of the neurons until the axons block the flow channel between the first well and the second well, the first well and the second well connected by the flow channel are disconnected, so that the second well can be filled with a culture medium different from that in the first well to perform culture.

Thus, in the culture vessel having a plurality of wells, the cell bodies of the neurons cultured in the first well and the tips of the axons extending from the cell bodies cultured in the first well toward the second well and the other cells such as skeletal muscle cells cultured in the second well and joined to the tips of the axons can be cultured under separate suitable culture medium conditions.

A second aspect of the present disclosure is a culture method of culturing neurons in a culture vessel having a plurality of wells, the culture method comprising: filling a first well and a second well included in the plurality of wells in the culture vessel with a culture medium for the neurons, and seeding the neurons in the first well, the first well and the second well communicating with each other through a flow channel; culturing axons of the neurons until the axons reach the second well through the flow channel and also block the flow channel; and thereafter removing the culture medium in the second well, filling the second well with a culture medium for culturing the axons, and culturing the axons.

By culturing the axons of the neurons until the axons block the flow channel between the first well and the second well, the first well and the second well connected by the flow channel are disconnected, so that the second well can be filled with a culture medium different from that in the first well to perform culture.

Thus, in the culture vessel having a plurality of wells, while culturing the cell bodies of the neurons in the first well under suitable culture medium conditions, the tips of the axons extending from the cell bodies of the neurons can be efficiently cultured in the second well under different culture medium conditions for promoting the growth of the axons.

In the first and second aspects, preferably, the first well has a first depression capable of storing a cell mass, at a bottom thereof, and a cell mass of the neurons is seeded in the first depression.

Thus, the cell mass is prevented from breaking, and the axons extending from the cell bodies of the neurons tend to extend in one direction together.

In the first and second aspects, the neurons may be central neurons or peripheral neurons. Examples of the central neurons include cells derived from glutamatergic nerves, dopaminergic nerves, and GABAergic nerves. Examples of the peripheral neurons include cells derived from motor nerves and sensory nerves. The cells may be directly collected from an animal, or be an established cell line thereof. The cells may be obtained by differentiating and culturing various stem cells.

A third aspect of the present disclosure is a culture vessel comprising a plurality of wells and configured to culture neurons or co-culture a plurality of types of cells including the neurons, wherein the plurality of wells include: a first well having a first depression capable of storing a cell mass, at a bottom thereof; and a second well communicating with the first well through a flow channel, and the flow channel has a maximum width of 20 µm to 100 µm in a direction orthogonal to a longitudinal direction thereof, and a depth of 20 µm to 100 µm.

By limiting the flow channel through which the wells communicate with each other to the foregoing ranges, the flow channel can be blocked with the axons of the neurons cultured in an appropriate number depending on the type of the neurons. Subsequently, the second well can be filled with a culture medium different from that in the first well, and cells (co-cultured cells) other than neurons can be seeded in the second well and cultured to join to the tips of the axons using the culture medium suitable for co-culture. In the case of not performing co-culture, too, it is possible to use respective different culture mediums suitable for culture in the well for culturing the cell bodies of neurons and in the well for culturing the tip parts of the axons extending from the cell bodies.

In the third aspect, preferably, the neurons are peripheral neurons, and the number of the peripheral neurons seeded in the first depression is 1 × 10⁴ to 4 × 10⁴, and the flow channel has a maximum cross-sectional area of 4 × 10⁻⁶ cm² to 1 × 10⁻⁴ cm². The peripheral neurons are preferably motor neurons.

In the case where the cells seeded are peripheral neurons, the flow channel can be blocked more accurately by limiting the number of cells and the cross-sectional area of the flow channel to the foregoing ranges.

In the third aspect, preferably, the neurons are central neurons, and the number of the central neurons seeded in the first depression is 4 × 10⁴ to 8 × 10⁴, and the flow channel has a maximum cross-sectional area of 4 × 10-⁶ cm² to 1 × 10⁻⁴ cm². The central neurons are preferably glutamatergic neurons.

In the case where the cells seeded are central neurons, the flow channel can be blocked more accurately by limiting the number of cells and the cross-sectional area of the flow channel to the foregoing ranges.

In the third aspect, the second well may have a second depression capable of storing a cell mass, at a bottom thereof.

Thus, in the case where a cell mass for co-culture is seeded in the second well, the cell mass is prevented from breaking, and culture can be performed in a cell mass state of high cell density.

A fourth aspect of the present disclosure is a culture vessel comprising a plurality of wells and configured to culture neurons or co-culture a plurality of types of cells including the neurons, wherein the plurality of wells include: a first well having a first depression capable of storing a cell mass, at a bottom thereof; and a second well communicating with the first well through a flow channel, one end of the flow channel is connected to the bottom of the first well, and an other end of the flow channel is connected to a bottom of the second well, and the flow channel has a maximum width of 20 µm to 120 µm in a direction orthogonal to a longitudinal direction thereof, a depth of 20 µm to 120 µm, and a length of 2 mm to 6 mm in the longitudinal direction.

By limiting the size and length of the flow channel to the specific ranges, mixing of the liquid in the first well and the liquid in the second well through the flow channel can be prevented even in a stage in which the flow channel is not filled with cells. Therefore, the second well can be filled with a culture medium different from that in the first well to perform culture.

Thus, in the culture vessel having a plurality of wells, the cell bodies of the neurons cultured in the first well and the tips of the axons extending from the cell bodies cultured in the first well toward the second well and the other cells such as skeletal muscle cells cultured in the second well and joined to the tips of the axons can be cultured under separate suitable culture medium conditions.

In the foregoing aspects, a water contact angle of at least a surface of a part to be in contact with the neurons may be 90° to 15°.

In this way, the neurons can be caused to adhere to the culture surface of the culture vessel more stably and cultured.

In the foregoing aspects, at least a surface of a part to be in contact with the neurons may be made of an alicyclic structure-containing polymer.

Since an alicyclic structure-containing polymer has high transparency, low autofluorescence (especially green), and low toxicity, the neurons can be cultured stably in a state in which observation and analysis in a culture process is easy.

A fifth aspect of the present disclosure is a culture method of co-culturing a plurality of types of cells including neurons using the culture vessel according to the fourth aspect, the culture method comprising: filling the first well in the culture vessel with a culture medium for the neurons, and filling the second well communicating with the first well through the flow channel with a culture medium for co-cultured cells to be co-cultured with the neurons; seeding the neurons in the first well; seeding the co-cultured cells in the second well; culturing axons of the neurons until the axons of the neurons reach the second well through the flow channel; and thereafter culturing the axons of the neurons together with the co-cultured cells in the second well.

By using the culture vessel in which the size and length of the flow channel are limited to the specific ranges, mixing of the liquid in the first well and the liquid in the second well through the flow channel can be prevented even in a state in which cells are not present in the flow channel. Therefore, the second well can be filled with a culture medium different from that in the first well to perform culture from the culture start stage.

A sixth aspect of the present disclosure is a culture method of culturing neurons using the culture vessel according to the fourth aspect, the culture method comprising: filling the first well in the culture vessel with a culture medium for the neurons, and filling the second well communicating with the first well through the flow channel with a culture medium for culturing axons; seeding the neurons in the first well; culturing axons of the neurons until the axons reach the second well through the flow channel; and thereafter culturing the axons in the second well.

By using the culture vessel in which the size and length of the flow channel are limited to the specific ranges, mixing of the liquid in the first well and the liquid in the second well through the flow channel can be prevented even in a state in which cells are not present in the flow channel. Therefore, the second well can be filled with a culture medium different from that in the first well to perform culture from the culture start stage.

### (Advantageous Effect)

It is thus possible to culture cells in each of a plurality of wells in a culture vessel under optimal culture conditions depending on the type of the cells cultured in the well.

### BRIEF DESCRIPTION OF THE DRAWINGS

In the accompanying drawings:
FIG. 1 is a plan view of a well body of a 1 × 8 strip well for a 96-well plate according to one of the disclosed embodiments;
FIG. 2 is a sectional view of the well body in FIG. 1;
FIG. 3 is a partial plan view of a member forming part of a well bottom that is joined to the well body in FIG. 1;
FIG. 4 is a sectional view of the member forming part of the well bottom in FIG. 3;
FIG. 5 is a partial plan view of a 1 × 8 strip well for a 96-well plate according to another one of the disclosed embodiments;
FIG. 6 is a partial plan view of a 96-well plate according to yet another one of the disclosed embodiments;
FIG. 7 is a partial plan view of a well body of a 1 × 8 strip well for a 96-well plate according to yet another one of the disclosed embodiments;
FIG. 8 is a plan view of a member forming part of a well bottom that is joined to the well body in FIG. 7;
FIG. 9 is a sectional view of the member forming part of the well bottom in FIG. 8;
FIG. 10 is a plan view of a 6-well plate according to yet another one of the disclosed embodiments;
FIG. 11 is a sectional view of the 6-well plate in FIG. 10; and
FIG. 12 is an inverted micrograph (× 40) of an axon bundle extending through a flow channel.

### DETAILED DESCRIPTION

Some of the disclosed embodiments will be described in detail below, with reference to the drawings.

A culture method and a culture vessel according to the present disclosure have a feature that, when co-culturing neurons and other cells or when culturing the cell bodies of neurons and the tips of their axons in different wells, a flow channel through which a first well and a second well communicate with each other in the culture vessel is blocked with the cultured axons of the neurons or the size and length of the flow channel through which the first well and the second well communicate with each other in the culture vessel are controlled to specific ranges, thus enabling culture using a different culture medium in the second well from a culture medium in the first well.

Hence, culture can be performed efficiently using, in each well, a different culture medium depending on the cells cultured in the well, with no need for a special process or member for blocking the flow channel.

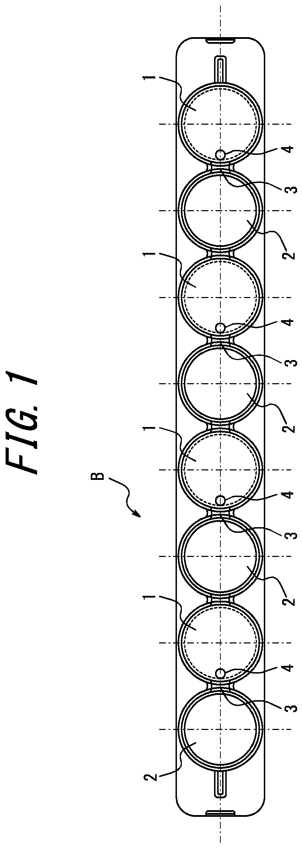

FIG. 1 is a plan view of a well body B of a 1 × 8 strip well for a 96-well plate according to one of the disclosed embodiments. As illustrated in FIG. 1, the well body B has a plurality of wells including a first well 1 and a second well 2, and has, on its bottom surface, positioning depressions 5 for joining the well body B to a member D forming part of a well bottom. The first well 1 has a first depression 4 for seeding a cell mass of neurons N. The bottom of the first depression 4 is open, and forms part of a flow channel 3. The flow channel 3 is defined by the first depression 4, the bottom surface of the well body B, and a groove on the upper surface of the member D forming part of the well bottom, as a result of the well body B being joined to the member D forming part of the well bottom.

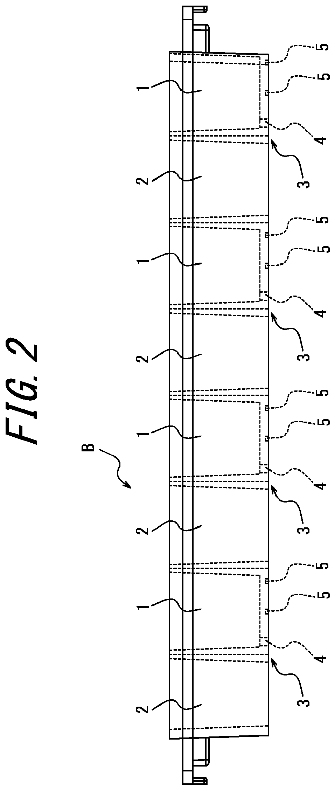

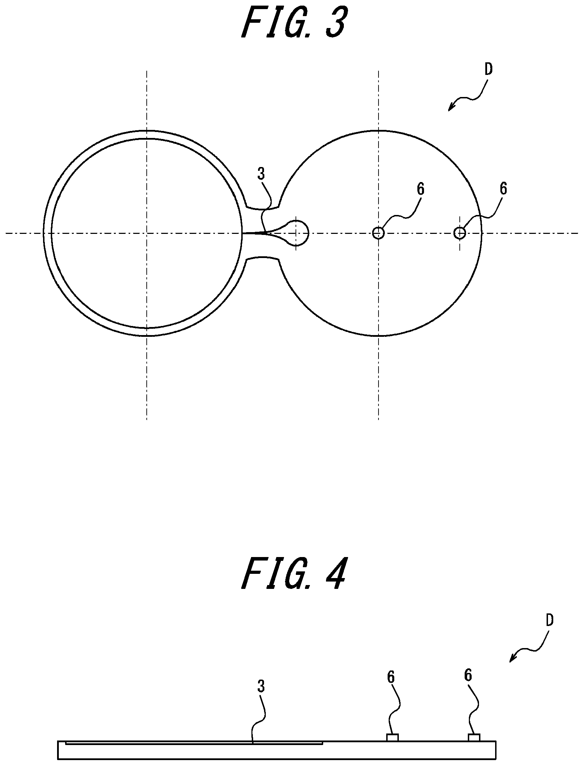

FIG. 2 is a sectional view of the well body B in FIG. 1. FIG. 3 is a partial plan view of the member D forming part of the well bottom, which is joined to the well body B in FIG. 1. The member D includes the groove portion of the flow channel 3 through which the first well 1 and the second well 2 communicate with each other, and positioning projections 6 for joining the member D to the well body B. The planar shape of the member D forming part of the well bottom conforms to the planar shape of the well body B in FIG. 1, although the planar shape of the member D is not limited to such. FIG. 4 is a sectional view of the member D forming part of the well bottom. As a result of joining the positioning depressions 5 of the well body B in FIGS. 1 and 2 and the positioning projections 6 of the member D forming part of the well bottom in FIGS. 3 and 4, a well strip having the flow channel 3 through which the first well 1 and the second well 2 communicate with each other is obtained. Since the member D forming part of the plate bottom portion in FIGS. 3 and 4 is made up of two wells, four members D each forming part of the well bottom are joined to one well body B. The presently disclosed techniques are, however, not limited to such, and one member D may have the same number of wells as one well body B and be joined to one well body B to obtain one well strip.

FIGS. 1 to 4 illustrate an example of a well plate (for example, model number: MS-8508M manufactured by Sumitomo Bakelite Co., Ltd.) of the same size as 96-well plates commonly used in analyzers, obtained by connecting twelve 1 × 8 well strips with a holder. The presently disclosed techniques are, however, not limited to such, and a well strip or well plate shape having any number of wells, such as 6 wells, 12 wells, 24 wells, or 48 wells, may be used as long as there are a plurality of wells. The size of the plate is preferably in conformance with the ANSI/SBS standard compatible with experimental equipment, and is, for example, equal to the size of commercially available 96-well plates of approximately 8.5 in length and 12.8 in width.

The culture vessel according to the present disclosure is not limited to a strip well and a well plate, and may have any structure as long as it includes two or more culture portions with a flow channel therebetween. For example, two dishes may be connected through a flow channel.

The material of the culture vessel according to the present disclosure may be a material used in typical culture vessels, such as polystyrene, polycarbonate, polyolefin, a cycloolefin polymer (COP), polyimide, any of fluorinated products thereof, polydimethylsiloxane (PDMS), or glass. A COP is preferable because it has low autofluorescence and is suitable for fluorescent observation.

In the culture vessel illustrated in FIGS. 1 to 4, the flow channel 3 through which the first well 1 and the second well 2 communicate with each other is defined by the groove formed on the upper surface of the member D forming part of the well bottom and the flat bottom surface and the first depression 4 of the well body B. The presently disclosed techniques are, however, not limited to such. For example, the flow channel 3 may be defined by a groove formed on the bottom surface of the well body B and the first depression 4 and the flat upper surface of the member D forming part of the well bottom. Alternatively, a groove may be formed on each of the bottom surface of the well body B and the upper surface of the member D forming part of the well bottom, and the bottom surface of the well body B and the upper surface of the member D may be joined to form one flow channel 3.

The cross-sectional shape of the groove forming the flow channel 3 in a direction orthogonal to its longitudinal direction may be rectangular, or semicircular with an arc-shaped bottom surface. The flow channel 3 formed may be rectangular, circular, oval, or the like.

Regarding the size of the flow channel 3, the maximum width of the flow channel in a direction orthogonal to its longitudinal direction is 20 µm to 100 µm, and the depth of the flow channel is 20 µm to 100 µm. Alternatively, the maximum width of the flow channel in a direction orthogonal to its longitudinal direction is 20 µm to 120 µm, the depth of the flow channel is 20 µm to 120 µm, and the length of the flow channel in the longitudinal direction is 2 mm to 6 mm. By limiting the flow channel through which the wells communicate with each other to such ranges, circulation of a culture medium between the wells is prevented, with it being possible to discard only the culture medium in the second well. Thus, the second well can be filled with a culture medium different from that in the first well to culture the tips of the axons or co-culture the axons and cells other than neurons in the second well. Preferably, the maximum width of the flow channel 3 in the direction orthogonal to the longitudinal direction is 20 µm to 80 µm and the depth of the flow channel is 20 µm to 80 µm, from the viewpoint of further enhancing the sealability.

A culture method according to one aspect of the present disclosure will be described below. The first well 1 is filled with a culture medium for neuron culture, and the second well 2 is filled with a culture medium suitable for culturing cells C to be co-cultured.

Neurons N made into a cell mass by culture are seeded in the first depression 4 formed at the bottom of the first well 1. The cells C to be co-cultured are seeded in the second well 2.

After performing culture until axons a extend from the cell bodies s of the seeded neurons N and reach the second well 2 through the flow channel 3, the tips of the extended axons a and the cells C are co-cultured in the second well 2 to join the axons a and the cells C to be co-cultured.

A culture method according to another aspect of the present disclosure will be described below. The first well 1 and the second well 2 are filled with a culture medium for neuron culture, and neurons N made into a cell mass by culture are seeded in the first depression 4 formed at the bottom of the first well 1. Culture is performed until axons a extend from the cell bodies s of the seeded neurons N, reach the second well 2 through the flow channel 3, and further block the flow channel 3. As a result of the extended axons a completely blocking the flow channel 3, the culture medium in the first well 1 and the culture medium in the second well 2 can be replaced independently of each other. Accordingly, only the culture medium in the second well 2 in which the tips of the extended axons a are cultured is discarded by suction or the like.

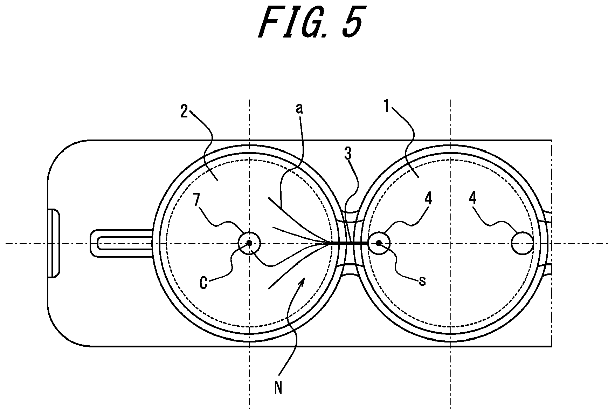

Subsequently, the second well 2 is filled with a culture medium suitable for culturing cells C to be co-cultured, and the cells C to be co-cultured are seeded and co-cultured, to join the axons a and the cells C to be co-cultured. As illustrated in FIG. 5, the second well 2 may have a depression (second depression 7) at the bottom, and the cells C to be co-cultured, which have been made into a cell mass, may be seeded in the second depression 7.

Alternatively, instead of filling the second well 2 with the culture medium suitable for culturing the cells C to be co-cultured, the second well 2 may be filled with a culture medium suitable for culturing the tips of the axons a, to continue the culture of the axons a alone.

Hence, neurotransmitters secreted from the axon terminals (presynaptic terminals), such as acetylcholine, glutamic acid, dopamine, serotonin, adrenaline, and noradrenaline, can be detected from the culture medium in the second well 2 using an enzyme reaction system, and used for neurotoxicity evaluation, drug discovery screening, diagnosis, and the like.

As a result of cell culture by co-culture through the foregoing process, a neural network is formed in the second well 2. By performing such a process in a plurality of sets of wells, a plurality of neural networks can be formed in the multiwell plate.

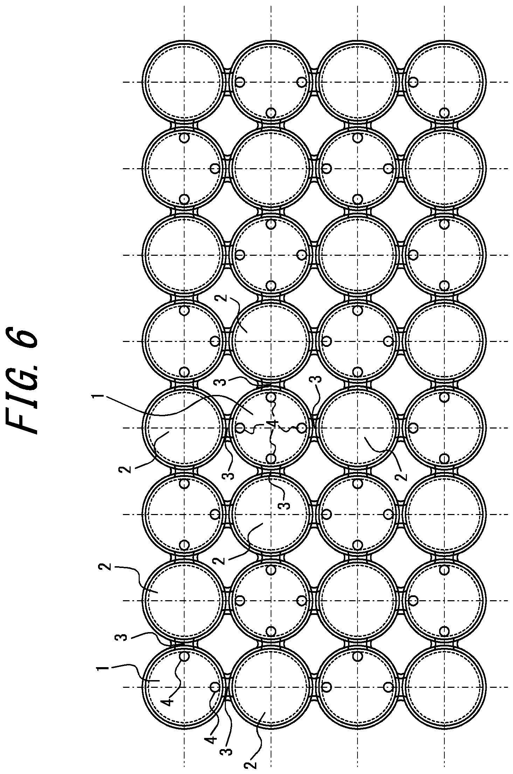

Specifically, in the case of performing co-culture, assuming one first well 1 and a maximum of four second wells 2 adjacent to the first well 1 as one set, a plate in which a flow channel 3 is formed between the first well 1 and each of the four second wells 2 at the maximum may be used, as illustrated in FIG. 6. A cell mass of neurons N is seeded in a first depression 4 corresponding to each flow channel 3, and the axons a of the neurons N are extended to the corresponding second well 2, thus blocking the four flow channels 3 at the maximum extending from the first well 1. The culture medium in each second well 2 is then sucked, and each second well 2 is filled with a culture medium suitable for culturing cells to be co-cultured in the second well 2. After this, the cells to be co-cultured are seeded in the second well 2. In this way, a maximum of four types of cells can be co-cultured with the axons a of the neurons N in one set of wells. By performing such a process in a plurality of sets of wells, a multiwell plate in which a plurality of neural networks are formed can be produced. The multiwell plate thus produced can be used for high-throughput drug screening.

FIG. 6 is a partial plan view of a 96-well plate, and illustrates 32 wells out of 96 wells. The number of wells is not limited to 96, and a multiwell plate with the same size as commercially available multiwell plates and the number of wells according to purpose, such as 6 wells, 12 wells, 24 wells, or 48 wells, may be produced and used.

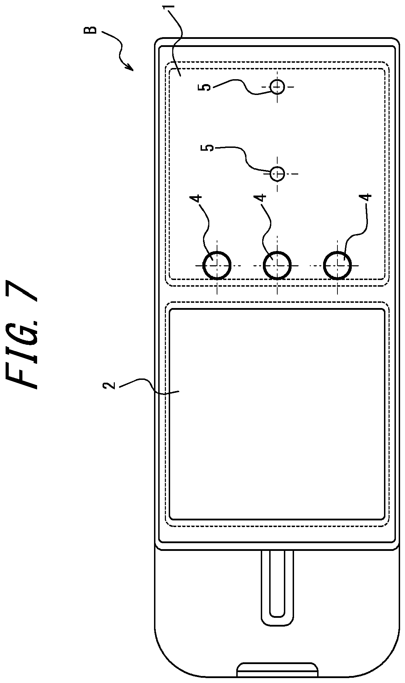

The planar shape of each well illustrated in FIGS. 1 to 6 is circular. The planar shape of each well is, however, not limited to such, and may be quadrilateral (rectangular) as illustrated in FIG. 7. In the case where the planar shape of each well is circular, the maximum number of flow channels 3 extending from one first well 1 is theoretically eight, for communication between wells with the same channel length. Due to the presence of inter-well walls necessary for design in manufacture, however, the maximum number of flow channels 3 extending from one well is typically four, as illustrated in FIG. 6. In the case where the planar shape of each well is quadrilateral, a plurality of flow channels 3 through which adjacent wells of one set of wells can communicate with each other can be formed with the same channel length. For example, three first depressions 4 are formed for each side of a quadrilateral first well 1 adjacent to a second well 2. If there are four second wells 2 adjacent to the first well 1, a maximum of twelve flow channels 3 extending from one first well 1 can be formed. Moreover, in the case where the planar shape of each well is quadrilateral, the length of the flow channel 3 can be shortened as compared with the case where the planar shape of each well is circular.

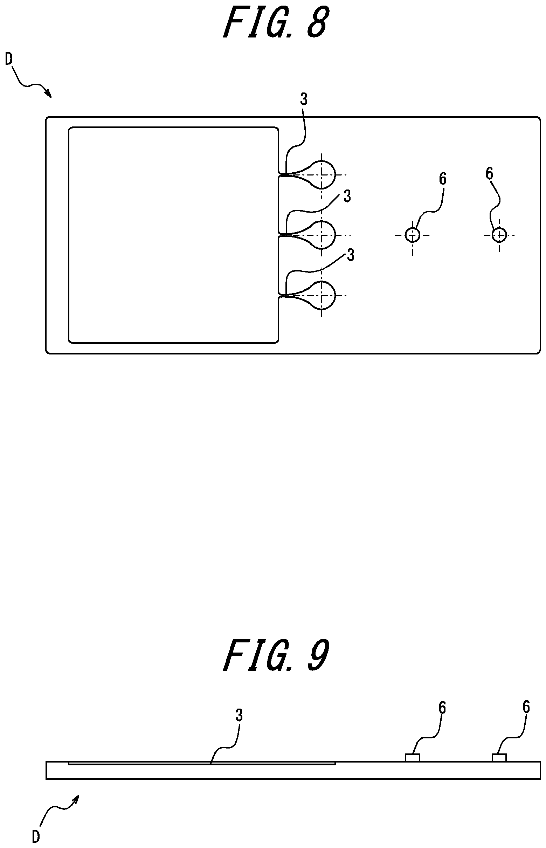

FIG. 7 is a partial plan view of a well body B of a 1 × 8 strip well for a 96-well plate according to another one of the disclosed embodiments. The well body B has a first well 1, a second well 2, first depressions 4, and positioning depressions 5. FIGS. 8 and 9 are respectively a plan view and a sectional view of a member D forming part of a well bottom, which is joined to the well body B in FIG. 7. The member D has grooves of flow channels 3 through which the first well 1 and the second well 2 communicate with each other, and positioning projections 6 for joining the member D to the well body B.

The embodiment illustrated in FIGS. 7 to 9 has the same structure as the strip well illustrated in FIGS. 1 to 4, but differs in that three flow channels 3 and three first depressions 4 are formed for one second well 2 from one first well 1. As a result of the planar shape of each well being quadrilateral, a plurality of flow channels 3 of the same length can be formed for one pair of a first well 1 and a second well 2. Further, with this well shape, the length of each flow channel 3 can be shortened as compared with the case where the planar shape of each well is circular. This can reduce the culture period for the axons a to reach the second well 2 and block the flow channel 3.

In this embodiment, three first depressions 4 and three flow channels 3 are formed per side of a rectangular well. The presently disclosed techniques are, however, not limited to such, and any number of first depressions 4 and flow channels 3 may be formed per side depending on the well size and the size of each first depression 4.

Each first depression 4 preferably has a diameter of 0.5 mm to 2 mm, from the viewpoint of easy placement of a cell mass.

The first depression 4 may have the same diameter or different diameters at its opening and bottom. The diameter at the opening is preferably greater than or equal to the diameter at the bottom, from the viewpoint of operability and more stable culture of a cell mass. Preferably, the first depression 4 is tapered, i.e. decreases in diameter, from the opening to the bottom, from the viewpoint of easier placement of a cell mass. More preferably, the first depression 4 is Y-shaped, that is, the first depression 4 is tapered from the opening to a midpoint in a direction toward the bottom and has the same diameter from the midpoint to the bottom.

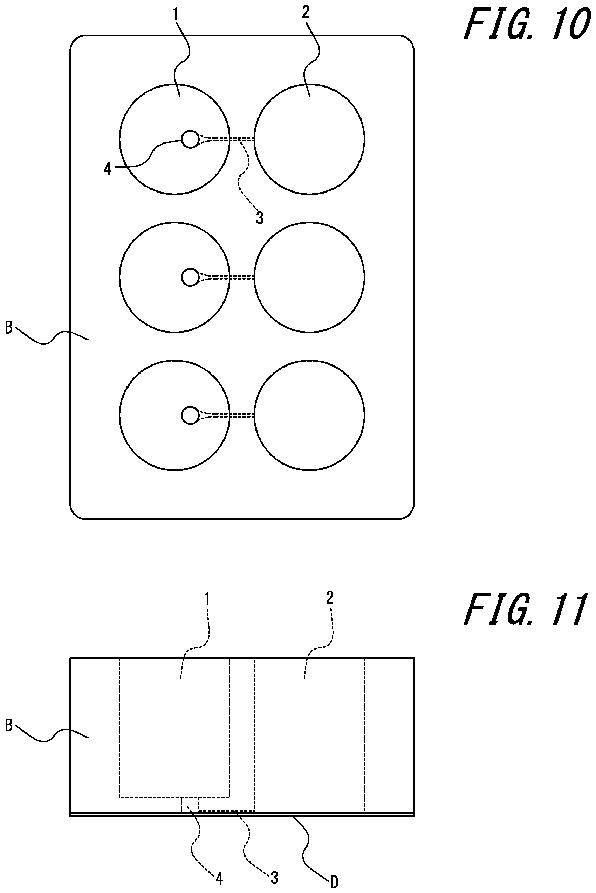

FIGS. 10 and 11 are respectively a plan view and a sectional view of a 6-well plate according to yet another one of the disclosed embodiments. The embodiment illustrated in FIGS. 10 and 11 has the same structure as the strip well illustrated in FIGS. 1 to 4, but differs in the following: While the groove forming part of the flow channel 3 is formed on the upper surface of the member D forming part of the well bottom in the strip well illustrated in FIGS. 1 to 4, the groove is formed on the lower surface of the well body B in this embodiment. With either groove forming method, the joint portion is on the bottom side of the flow channel, so that the proliferation of neurons N is not hindered by protrusion of the plate forming material into the flow channel 3 when joining the well body B and the member D forming part of the well bottom.

The size of the flow channel 3 is approximately the same as the size (thickness) of an axon bundle formed by bundling a plurality of axons a of neurons N cultured. The size of the axon bundle depends on the type and number of neurons N. For motor neurons, in the case where the number of cells is 1 × 10⁴ to 2 × 10⁴, the size of the axon bundle is approximately 80 µm. For glutamatergic neurons, in the case where the number of cells is 3 × 10⁴ to 5 × 10⁴, the size of the axon bundle is approximately 80 µm. The number of cells cultured is adjusted so that the size of the axon bundle will be approximately the same as the size of the entrance portion of the flow channel 3. Since the speed of extension differs among axons, the size of the axon bundle tends to decrease toward the tip of the axon bundle. However, by adjusting the number of cells in this way, the flow channel 3 can be blocked with the axon bundle at its entrance portion.

In view of the above, the size of the flow channel 3 in a co-culture vessel according to one aspect of the present disclosure is such that the maximum width of the flow channel 3 in the direction orthogonal to the longitudinal direction is 20 µm to 100 µm, and the depth of the flow channel is 20 µm to 100 µm.

From the viewpoint of enhancing the sealability of the flow channel more reliably, in the case where the cells seeded are peripheral neurons, it is preferable that the number of peripheral neurons seeded in the first depression 4 is 1 × 10⁴ to 4 × 10⁴ and the cross-sectional area of the flow channel 3 is 4 × 10-⁶ cm² to 1 × 10⁻⁴ cm². In the case where the cells seeded are central neurons, it is preferable that the number of central neurons seeded in the first depression 4 is 4 × 10⁴ to 8 × 10⁴ and the cross-sectional area of the flow channel 3 is 4 × 10⁻⁶ cm² to 1 × 10⁻⁴ cm².

Examples of the cells C to be co-cultured include skeletal muscle cells and skin cells, without being limited thereto. Instead of or together with the cells C to be co-cultured, an artificial material such as microbeads covered with Lrp4 described in Yumoto et al., "Lrp4 is a retrograde signal for presynaptic differentiation at neuromuscular synapses", Nature, 489, 438-442 (2012) may be used in co-culture. In the case of using such an artificial material in co-culture, the culture medium may be the same as or different from the culture medium for neurons to be placed in the first well.

The neurons to be cultured may be central neurons or peripheral neurons. Examples of the central neurons include cells derived from glutamatergic nerves, dopaminergic nerves, and GABAergic nerves. Examples of the peripheral neurons include cells derived from motor nerves and sensory nerves. The cells may be directly collected from an animal, or be an established cell line thereof. The cells may be obtained by differentiating and culturing various stem cells.

The size (thickness) of the axon bundle extending from the neurons depends on the type and number of neurons. For motor neurons, in the case where the number of cells is 1 × 10⁴ to 2 × 10⁴, the size of the axon bundle is approximately 80 µm. For glutamatergic neurons, in the case where the number of cells is 3 × 10⁴ to 5 × 10⁴, the size of the axon bundle is approximately 80 µm.

In a culture vessel according to another aspect of the present disclosure, the size of the flow channel through which the first well and the second well communicate with each other is such that the maximum width of the flow channel in the direction orthogonal to the longitudinal direction is 20 µm to 120 µm, the depth of the flow channel is 20 µm to 120 µm, and the length of the flow channel in the longitudinal direction is 2 mm to 6 mm.

If any of the width and depth of the flow channel is less than 20 µm, it is difficult to form the flow channel, and the axons may be unable to extend through the flow channel. If any of the width and depth of the flow channel is more than 120 µm, the culture medium in the first well and the culture medium in the second well tend to mix through the flow channel.

If the length of the flow channel in the longitudinal direction is less than 2 mm, the culture medium in the first well and the culture medium in the second well tend to mix through the flow channel. If the length of the flow channel in the longitudinal direction is more than 6 mm, the axons may be unable to reach the second well through the flow channel.

By limiting the size and length of the flow channel to the foregoing ranges, mixing of the liquid in the first well and the liquid in the second well through the flow channel can be prevented stably even in a stage in which no cells are present in the flow channel. Therefore, the second well can be filled with a culture medium different from that in the first well to perform culture from culture start. This makes it possible to prepare for culture of the tips of axons or co-culture of axons and cells other than neurons in the second well beforehand.

To fill the flow channel with the thickness of the axons and allow the tips of the axons to reach the second well, it is preferable that the maximum width of the flow channel in the direction orthogonal to the longitudinal direction is 60 µm to 80 µm, the depth of the flow channel is 60 µm to 80 µm, and the length of the flow channel in the longitudinal direction is 2 mm to 3 mm.

In a culture vessel according to the foregoing aspects of the present disclosure, at least the water contact angle of the surface of the part to be in contact with the neurons is preferably 90° to 15°. By limiting the water contact angle to this range, the neurons can be adhered to the culture surface of the culture vessel more stably and cultured. The water contact angle is more preferably 90° to 60°, in order to ensure a moderate (i.e. not excessively high or low) adhesion strength of the cells. For example, the water contact angle can be limited to the foregoing range by surface modification treatment such as atmospheric pressure plasma treatment, reduced pressure plasma treatment, vacuum ultraviolet treatment, corona treatment, or ozone treatment.

The water contact angle herein is calculated as follows: Using a fully automatic contact angle meter (LCD-400S manufactured by Kyowa Interface Science Co., Ltd.), the radius r and height h of a liquid droplet are measured at each of a total of five measurement points of a sample obtained by cutting the bottom surface of a culture vessel (well) with a Φ30 mm circle cutter, i.e. the center of the sample and the four vertices of a square centered at the center of the sample and having a side length of 20 mm. θ is then calculated according to tanθ1 = h/r,θ = 2θ₁ → θ = 2arctan(h/r), and taken to be the water contact angle (θ/2 method).

The material of the culture vessel according to the foregoing aspects of the present disclosure may be a material used in typical culture vessels, such as polystyrene, polycarbonate, polyolefin, a cycloolefin polymer (COP), polyimide, any of fluorinated products thereof, polydimethylsiloxane (PDMS), or glass.

The material of the culture vessel is preferably an alicyclic structure-containing polymer because it has low autofluorescence of green and is suitable for fluorescent observation, has high transparency, and has low toxicity. At least the surface of the part to be in contact with the neurons is preferably made of an alicyclic structure-containing polymer.

The alicyclic structure-containing polymer is a resin having an alicyclic structure in the main chain and/or side chain. The alicyclic structure-containing polymer preferably contains an alicyclic structure in the main chain, from the viewpoint of the mechanical strength, the heat resistance, and the like. The alicyclic structure-containing polymer more preferably does not contain a polar group, from the viewpoint of the differentiation induction efficiency. The "polar group" herein denotes a polar atomic group. Examples of the polar group include an amino group, a carboxyl group, a hydroxyl group, and an acid anhydride group.

Examples of the alicyclic structure include a saturated cyclic hydrocarbon (cycloalkane) structure and an unsaturated cyclic hydrocarbon (cycloalkene) structure. From the viewpoint of the mechanical strength, the heat resistance, and the like, a cycloalkane structure and a cycloalkene structure are preferable, and a cycloalkane structure is most preferable.

The number of carbon atoms in the alicyclic structure is not limited, but is typically 4 to 30, preferably 5 to 20, and more preferably 5 to 15. The number of carbon atoms in the alicyclic structure within such range is favorable because the mechanical strength, the heat resistance, and the formability are well-balanced.

The proportion of the repeating unit having the alicyclic structure in the alicyclic structure-containing polymer is selected as appropriate depending on the intended use, but is typically 30 wt% or more, preferably 50 wt% or more, and more preferably 70 wt% or more. If the proportion of the repeating unit having the alicyclic structure in the alicyclic structure-containing polymer is excessively low, the heat resistance is poor, which is not desirable. The balance other than the repeating unit having the alicyclic structure in the alicyclic structure-containing polymer is not limited, and is selected as appropriate depending on the intended use.

Specific examples of the alicyclic structure-containing polymer include (1) a norbornene-based polymer, (2) a monocyclic cyclic olefin-based polymer, (3) a cyclic conjugated diene-based polymer, (4) a vinyl alicyclic hydrocarbon-based polymer, and hydrides of (1) to (4). Of these, a norbornene-based polymer and a hydride thereof are preferable from the viewpoint of the heat resistance, the mechanical strength, and the like.

### (1) Norbornene-based polymer

A norbornene-based polymer is obtained by polymerizing a norbornene-based monomer which is a monomer having a norbornene skeleton, and is roughly divided into a norbornene-based polymer obtained by ring-opening polymerization and a norbornene-based polymer obtained by addition polymerization.

Examples of the norbornene-based polymer obtained by ring-opening polymerization (cycloolefin polymer (COP)) include a ring-opening polymer of a norbornene-based monomer, a ring-opening polymer of a norbornene-based monomer and another monomer ring-opening copolymerizable with the norbornene-based monomer, and hydrides thereof. Examples of the norbornene-based polymer obtained by addition polymerization (cycloolefin copolymer (COC)) include an addition polymer of a norbornene-based monomer and an addition polymer of a norbornene-based monomer and another monomer copolymerizable with the norbornene-based monomer. Of these, a ring-opening polymer hydride of a norbornene-based monomer is preferable from the viewpoint of the heat resistance, the mechanical strength, and the like.

Examples of the norbornene-based monomer that can be used in the synthesis of the norbornene-based polymer include bicyclic monomers such as bicyclo[2.2.1]hepta-2-ene (common name: norbornene), 5-methyl-bicyclo[2.2.1]hepta-2-ene, 5,5-dimethyl-bicyclo[2.2.1]hepta-2-ene, 5-ethyl-bicyclo[2.2.1]hepta-2-ene, 5-ethylidene-bicyclo[2.2.1]hepta-2-ene, 5-vinyl-bicyclo[2.2.1]hepta-2-ene, 5-propenylbicyclo[2.2.1]hepta-2-ene, 5-methoxycarbonyl-bicyclo[2.2.1]hepta-2-ene, 5-cyanobicyclo[2.2.1]hepta-2-ene, and 5-methyl-5-methoxycarbonyl-bicyclo[2.2.1]hepta-2-ene; tricyclic monomers such as tricyclo[4.3.0^{1,6}.1^{2,5}]deca-3,7-diene (common name: dicyclopentadiene), 2-methyldicyclopentadiene, 2,3-dimethyldicyclopentadiene, and 2,3-dihydroxydicyclopentadiene; and tetracyclic monomers such as tetracyclo[4.4.0.1^{2,5}.1^{7,10}]-3-dodecene (tetracyclododecene), tetracyclo[4.4.0.1^{2,5}.1^{7,10}]-3-dodecene, 8-methyltetracyclo[4.4.0.1^{2,5}.1^{7,10}]-3-dodecene, 8-ethyltetracyclo[4.4.0.1^{2,5}.1^{7,10}]-3-dodecene, 8-ethylidenetetracyclo[4.4.0.1^{2,5}.1^{7,10}]-3-dodecene, 8,9-dimethyltetracyclo[4.4.0.1^{2,5}.1^{7,10}]-3-dodecene, 8-ethyl-9-methyltetracyclo[4.4.0.1^{2,5}.1^{7,10}]-3-dodecene, 8-ethylidene-9-methyltetracyclo[4.4.0.1^{2,5}.1^{7,10}]-3-dodecene, 8-methyl-8-carboxymethyltetracyclo[4.4.0.1^{2,5}.1^{7,10}]-3-dodecene, 7,8-benzotricyclo[4.3.0.1^{2,5}]deca-3-ene (common name: methanotetrahydrofluorene, also called 1,4-methano-1,4,4a,9a-tetrahydrofluorene), 1,4-methano-8-methyl-1,4,4a,9a-tetrahydrofluorene, 1,4-methano-8-chloro-1,4,4a,9a-tetrahydrofluorene, and 1,4-methano-8-bromo-1,4,4a,9a-tetrahydrofluorene.

Examples of another monomer ring-opening copolymerizable with the norbornene-based monomer include monocyclic cycloolefin-based monomers such as cyclohexene, cycloheptene, cyclooctene, 1,4-cyclohexadiene, 1,5-cyclooctadiene, 1,5-cyclodecadiene, 1,5,9-cyclododecatriene, and 1,5,9,13-cyclohexadecatetraene.

These monomers may have one or more substituents. Examples of the substituents include an alkyl group, an alkylene group, an aryl group, a silyl group, an alkoxycarbonyl group, and an alkylidene group.

Examples of another monomer addition copolymerizable with the norbornene-based monomer include α-olefin-based monomers having a carbon number of 2 to 20 such as ethylene, propylene, 1-butene, 1-pentene, and 1-hexene; cycloolefin-based monomers such as cyclobutene, cyclopentene, cyclohexene, cyclooctene, and tetracyclo[9.2.1.0^{2,10}.0^{3,8}]tetradeca-3,5,7,12-tetraene (also called 3a,5,6,7a-tetrahydro-4,7-methano-1H-indene); and nonconjugated diene-based monomers such as 1,4-hexadiene, 4-methyl-1,4-hexadiene, 5-methyl-1,4-hexadiene, and 1,7-octadiene.

Of these, α-olefin-based monomers are preferable and ethylene is more preferable as another monomer addition copolymerizable with the norbornene-based monomer.

These monomers may have one or more substituents. Examples of the substituents include an alkyl group, an alkylene group, an aryl group, a silyl group, an alkoxycarbonyl group, and an alkylidene group.

A ring-opening polymer of a norbornene-based monomer or a ring-opening polymer of a norbornene-based monomer and another monomer ring-opening copolymerizable with the norbornene-based monomer can be obtained by polymerizing a monomer component in the presence of a known ring-opening polymerization catalyst. Examples of the ring-opening polymerization catalyst include a catalyst composed of: a halide of a metal such as ruthenium or osmium; nitrate or an acetylacetone compound; and a reductant, and a catalyst composed of: a halide of a metal such as titanium, zirconium, tungsten, or molybdenum or an acetylacetone compound; and an organic aluminum compound.

Typically, a ring-opening polymer hydride of a norbornene-based monomer can be obtained by adding a known hydrogenation catalyst containing a transition metal such as nickel or palladium to a polymerization solution of the ring-opening polymer to hydrogenate a carbon-carbon unsaturated bond.

An addition polymer of a norbornene-based monomer or an addition polymer of a norbornene-based monomer and another monomer copolymerizable with the norbornene-based monomer can be obtained by polymerizing a monomer component in the presence of a known addition polymerization catalyst. Examples of the addition polymerization catalyst include a catalyst composed of: titanium, zirconium, or a vanadium compound; and an organic aluminum compound.

### (2) Monocyclic cyclic olefin-based polymer

Examples of the monocyclic cyclic olefin-based polymer include addition polymers of monocyclic cyclic olefin-based monomers such as cyclohexene, cycloheptene, and cyclooctene.

### (3) Cyclic conjugated diene-based polymer

Examples of the cyclic conjugated diene-based polymer include polymers obtained by 1,2- or 1,4-addition polymerization of cyclic conjugated diene-based monomers such as cyclopentadiene and cyclohexadiene, and hydrides thereof.

### (4) Vinyl alicyclic hydrocarbon polymer

Examples of the vinyl alicyclic hydrocarbon polymer include polymers of vinyl alicyclic hydrocarbon-based monomers such as vinyl cyclohexene and vinyl cyclohexane and hydrides thereof; and hydrides of aromatic ring portions of polymers of vinyl aromatic monomers such as styrene and α-methylstyrene. The vinyl alicyclic hydrocarbon polymer may be a copolymer of any of these monomers and another monomer copolymerizable with the monomer.

The molecular weight of the alicyclic structure-containing polymer is not limited, but is typically 5,000 or more, preferably 5,000 to 500,000, more preferably 8,000 to 200,000, and particularly preferably 10,000 to 100,000 in terms of the polyisoprene-equivalent weight average molecular weight measured by gel permeation chromatography in a cyclohexane solution (a toluene solution in the case where the polymer does not dissolve). The weight average molecular weight within such range is favorable because the mechanical strength and the formability are well-balanced.

The glass transition temperature of the alicyclic structure-containing polymer is selected as appropriate depending on the intended use, but is typically 50 °C to 300 °C, preferably 80 °C to 280 °C, particularly preferably 90 °C to 250 °C, and further preferably 90 °C to 200 °C. The glass transition temperature within such range is favorable because the heat resistance and the formability are well-balanced.

Herein, the glass transition temperature of the alicyclic structure-containing polymer is measured in accordance with JIS K 7121.

The foregoing alicyclic structure-containing polymers may be used alone or in combination of two or more.

Blending agents typically used in thermoplastic resin materials may be added to the alicyclic structure-containing polymer, in amounts typically used. Examples of the blending agents include a soft polymer, an antioxidant, an ultraviolet absorber, a light stabilizer, a near infrared absorber, a mold release agent, a colorant such as a dye or a pigment, a plasticizer, an antistatic agent, and a fluorescent whitening agent.

The alicyclic structure-containing polymer may be mixed with one or more polymers (hereafter simply referred to as "other polymers") other than the soft polymer. The amount of the other polymers mixed with the alicyclic structure-containing polymer is typically 200 mass parts or less, preferably 150 mass parts or less, and more preferably 100 mass parts or less with respect to 100 mass parts of the alicyclic structure-containing polymer.

If the proportions of the blending agents and the other polymers added to the alicyclic structure-containing polymer are excessively high, floating of cells is hindered. It is therefore preferable to add the blending agents and the other polymers in such ranges that will not impair the properties of the alicyclic structure-containing polymer.

The method of mixing the alicyclic structure-containing polymer with the blending agents and the other polymers is not limited as long as the blending agents are sufficiently dispersed in the polymer. The blending order is not limited, either. Examples of the blending method include a method of kneading resins in a molten state using a mixer, a uniaxial kneader, a biaxial kneader, a roll, a Brabender, an extruder, or the like, and a method of dissolving and dispersing resins in an appropriate solvent and then removing the solvent by a solidification method, a casting method, or a direct drying method.

In the case of using a biaxial kneader, after kneading, the kneaded material is usually extruded into a rod shape in a molten state, cut to an appropriate length with a strand cutter, and pelletized.

As the method of forming the culture vessel used in the present disclosure, any method may be selected depending on the desired shape of the culture vessel. Examples of the method include injection molding, extrusion, casting, inflation molding, blow molding, vacuum forming, press forming, compression molding, rotational molding, calendaring, roll forming, cutting, and spinning. Two or more of these forming methods may be used in combination.

The culture vessel used in the present disclosure is preferably subjected to sterilization treatment.

The sterilization treatment method is not limited, and may be selected from the methods commonly used in the medical field depending on the shape of the formed product and the cells used. Examples of such methods include heating methods such as a high-pressure steam method and a dry heat method; radiation methods that involve irradiation with radiation rays such as gamma rays or electron beams and irradiation methods that involve high-frequency irradiation; gas methods that involve contact with gases such as ethylene oxide gas (EOG); and filtration methods using sterilization filters.

### EXAMPLES

The presently disclosed techniques will be described in detail below by way of examples, although the presently disclosed techniques are not limited to these examples.

### (Example 1-1)

The bottom of each well of a culture vessel according to the present disclosure in a 1 × 8 strip well shape made of a cycloolefin polymer (ZEONEX^{®}690R manufactured by Zeon Corporation (ZEONEX is a registered trademark in Japan, other countries, or both)) and having four pairs of first wells 1 and second wells 2 as illustrated in FIG. 1 was coated with a coating liquid (iMatrix^{®}-211 manufactured by Nippi, Inc. (iMatrix is a registered trademark in Japan, other countries, or both)). After this, 400 µl/well of a culture medium for neuron culture (model number: ST-05790 manufactured by Veritas Corporation) heated to 37 °C was added into each well. One cell mass (sphere) of 2 × 10⁴ motor neurons (iCell^{®} Motor Neuron manufactured by FUJIFILM Cellular Dynamics, Inc. (iCell is a registered trademark in Japan, other countries, or both)) cultured in a 96-well plate was seeded in the first depression 4 of 1 mm in diameter formed at the bottom of each first well 1. After making sure that the cell mass was placed in the first depression 4 using a microscope, the culture vessel was left in a 37 °C 5 % CO₂ incubator to perform culture. The culture medium was replaced every three or four days. Specifically, the culture medium was gently sucked and discarded using a 1000 µ pipette, and 400 µl/well of the new culture medium for neuron culture was added into each well.

The culture was continued until the axons extended from the motor neurons placed in the first depression 4 and reached the second well 2 through the flow channel 3 having a width of 80 µm in the direction orthogonal to the longitudinal direction and a depth of 80 µm and the axon bundle formed by bundling the axons completely blocked the flow channel 3. After this, the culture medium in the second well 2 was completely sucked and removed, and 400 µl/well of a culture medium (model number: C-23160 manufactured by Takara Bio Inc.) for culturing human skeletal muscle cells (model number: C-12530 manufactured by Takara Bio Inc.) to be co-cultured with the axons of the neurons was added into each second well 2. 14400 human skeletal muscle cells were then seeded in the second well 2, and co-cultured with the axons of the neurons to join the axons and the human skeletal muscle cells.

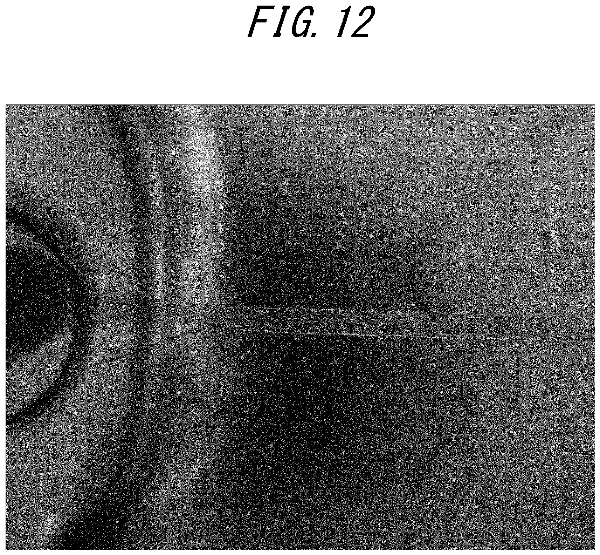

Whether the axon bundle completely blocked the flow channel 3 was determined by microscopic observation. As illustrated in the micrograph in FIG. 12, the axon bundle filled the flow channel 3. Even when the culture medium in the second well 2 was sucked, the culture medium in the first well 1 did not flow into the second well 2 from the flow channel 3. This indicates that the axon bundle completely blocked the flow channel 3.

### (Example 1-2)

The same culture process as in Example 1-1 was performed, except that the neurons to be cultured were glutamatergic neurons, the material of the culture vessel according to the present disclosure in a 1 × 8 strip well shape was polydimethylsiloxane (model number: SILPOT 184 W/C manufactured by Dow Toray Co., Ltd.), and co-cultured cells are not used. Specifically, 6 × 10⁴ glutamatergic neurons (iCell^{®} Gluta Neuron manufactured by FUJIFILM Cellular Dynamics, Inc.) were cultured using a culture medium for neuron culture (model number: ST-05790 manufactured by Veritas Corporation).

As in Example 1-1, the axon bundle filled and blocked the flow channel 3. Accordingly, even when the culture medium in the second well 2 was sucked, the culture medium in the first well 1 did not flow into the second well 2 from the flow channel 3. This indicates that the axon bundle completely blocked the flow channel 3.

After continuing the culture until the axon bundle formed by bundling the axons of the glutamatergic neurons completely blocked the flow channel 3 as described above, the culture medium in the second well 2 was completely sucked and removed. Glia-conditioned medium (product number: SBMBX9501D-3A manufactured by KAC Co., Ltd.) to which 100-fold diluted N2 Supplement (product number: 17502048 manufactured by Thermo Fisher Scientific, Inc.) and 50-fold diluted B27 Supplement (product number: 17504044 manufactured by Thermo Fisher Scientific, Inc.) had been added was then added and the culture was further continued. Consequently, secretion of glutamic acid which is a neurotransmitter secreted from axon terminals was observed from the culture medium in the second well 2 by examining the change in absorption of coenzyme using glutamate dehydrogenase.

### (Example 2-1)

A culture vessel composed of a well body B made of PDMS (product name: UV Cure Liquid Silicone Rubber, product number: KER-4690-A/B manufactured by Shin-Etsu Chemical Co., Ltd., water contact angle: 90°) and having four pairs of first wells 1 and second wells 2 as illustrated in FIG. 1 and a member D made of glass (product name: Borosilicate Glass manufactured by AGC Techno Glass Co., Ltd.) and forming part of the well bottom was used. The flow channel 3 formed between each pair of first well 1 and second well 2 was rectangular, and had a width of 120 µm in the direction orthogonal to the longitudinal direction, a depth of 120 µm, and a length of 2 mm in the longitudinal direction (i.e., distance between wells).

400 µl/well of a culture medium for neuron culture (product number: ST-05790 manufactured by Veritas Corporation) was added into each first well 1, and 400 µl/well of pure water was added into the second well 2 communicating with the first well 1 through the flow channel 3. It was visually confirmed that the culture medium for neuron culture in the first well 1 and the pure water in the second well 2 did not mix through the flow channel 3 even after 24 hours.

Subsequently, the culture medium for neuron culture in the first well 1 was gently sucked and discarded using a 1000 µ pipette, and 400 µl/well of a culture medium for neuron culture (model number: ST-05790 manufactured by Veritas Corporation) heated to 37 °C was added into each well. One cell mass (sphere) of 2 × 10⁴ motor neurons (iCell^{®} Motor Neuron manufactured by FUJIFILM Cellular Dynamics, Inc.) cultured in a 96-well plate was seeded in the first depression 4 of 1 mm in diameter formed at the bottom of each first well 1, and a microscope was used to make sure that the cell mass was placed in the first depression 4. Following this, the pure water in the second well 2 was gently sucked and discarded using a 1000 µ pipette, 400 µl/well of a culture medium (model number: C-23160 manufactured by Takara Bio Inc.) for culturing human skeletal muscle cells (model number: C-12530 manufactured by Takara Bio Inc.) to be co-cultured with the axons of the neurons was added into the second well 2. 14400 human skeletal muscle cells were then seeded in the second well 2. The culture vessel was left in a 37 °C 5 % CO₂ incubator to perform culture. The culture medium was replaced every three or four days. Specifically, the culture medium was gently sucked and discarded using a 1000 µ pipette, and 400 µl/well of the new culture medium was added into each well.

The culture was continued until the axons a extended from the motor neurons placed in the first depression 4 and reached the second well 2 through the flow channel 3 as illustrated in FIG. 12. Further, the human skeletal muscle cells were co-cultured with the axons of the neurons to join the axons and the human skeletal muscle cells.

The co-culture was successfully performed without mixing of the culture medium for neuron culture in the first well 1 and the culture medium for human skeletal muscle cell culture in the second well 2. Even in the case where the motor neurons are cultured alone, the culture medium for neuron culture in the first well 1 and the culture medium for culture of axons a in the second well 2 did not mix during a culture period of about one week.

### (Example 2-2)

The same test and cell culture process as in Example 2-1 were performed, except that the depth of the flow channel 3 in the culture vessel was 20 µm.

As in Example 2-1, it was confirmed that the culture medium for neuron culture in the first well 1 and the pure water in the second well 2 did not mix through the flow channel 3 even after 24 hours. Moreover, the co-culture was successfully performed without mixing of the culture medium for neuron culture in the first well 1 and the culture medium for human skeletal muscle cell culture in the second well 2.

### (Example 2-3)

The same test and cell culture process as in Example 2-1 were performed, except that the width of the flow channel 3 in the direction orthogonal to the longitudinal direction of the flow channel 3 in the culture vessel was 20 µm.

As in Example 2-1, it was confirmed that the culture medium for neuron culture in the first well 1 and the pure water in the second well 2 did not mix through the flow channel 3 even after 24 hours. Moreover, the co-culture was successfully performed without mixing of the culture medium for neuron culture in the first well 1 and the culture medium for human skeletal muscle cell culture in the second well 2.

### (Example 2-4)

The same test and cell culture process as in Example 2-1 were performed, except that the culture vessel was made of a COP (product name: ZEONEX, product number: 690R manufactured by Zeon Corporation), the width of the flow channel 3 in the direction orthogonal to the longitudinal direction was 80 µm, the depth of the flow channel 3 was 80 µm, and the water contact angle was 80°.

As in Example 2-1, it was confirmed that the culture medium for neuron culture in the first well 1 and the pure water in the second well 2 did not mix through the flow channel 3 even after 24 hours. Moreover, the co-culture was successfully performed without mixing of the culture medium for neuron culture in the first well 1 and the culture medium for human skeletal muscle cell culture in the second well 2.

### (Example 2-5)

The same test and cell culture process as in Example 2-1 were performed, except that the culture vessel was made of a COP, the width of the flow channel 3 in the direction orthogonal to the longitudinal direction was 80 µm, the depth of the flow channel 3 was 20 µm, and the water contact angle was 80°.

As in Example 2-1, it was confirmed that the culture medium for neuron culture in the first well 1 and the pure water in the second well 2 did not mix through the flow channel 3 even after 24 hours. Moreover, the co-culture was successfully performed without mixing of the culture medium for neuron culture in the first well 1 and the culture medium for human skeletal muscle cell culture in the second well 2.

### (Example 2-6)

The same test and cell culture process as in Example 2-1 were performed, except that the culture vessel was made of a COP, the width of the flow channel 3 in the direction orthogonal to the longitudinal direction was 20 µm, the depth of the flow channel 3 was 80 µm, and the water contact angle was 80°.

As in Example 2-1, it was confirmed that the culture medium for neuron culture in the first well 1 and the pure water in the second well 2 did not mix through the flow channel 3 even after 24 hours. Moreover, the co-culture was successfully performed without mixing of the culture medium for neuron culture in the first well 1 and the culture medium for human skeletal muscle cell culture in the second well 2.

### (Example 2-7)

The same test and cell culture process as in Example 2-1 were performed, except that the culture vessel was made of a COP and the length of the flow channel 3 in the longitudinal direction was 6 mm.

As in Example 2-1, it was confirmed that the culture medium for neuron culture in the first well 1 and the pure water in the second well 2 did not mix through the flow channel 3 even after 24 hours. Moreover, the co-culture was successfully performed without mixing of the culture medium for neuron culture in the first well 1 and the culture medium for human skeletal muscle cell culture in the second well 2.

### (Example 2-8)

The same test and cell culture process as in Example 2-1 were performed, except that the culture vessel was made of polystyrene (PS) (product name: Toyo Styrol GP grade: MW1C manufactured by Toyo Styrene Co., Ltd.), the width of the flow channel 3 in the direction orthogonal to the longitudinal direction was 60 µm, the depth of the flow channel 3 was 60 µm, and the water contact angle was 40°.

As in Example 2-1, it was confirmed that the culture medium for neuron culture in the first well 1 and the pure water in the second well 2 did not mix through the flow channel 3 even after 24 hours. Moreover, the co-culture was successfully performed without mixing of the culture medium for neuron culture in the first well 1 and the culture medium for human skeletal muscle cell culture in the second well 2.

### (Comparative Example 1-1)

The same culture process as in Example 1-1 was performed, except that the number of motor neurons cultured was different from Example 1-1. Specifically, the number of motor neurons cultured in the first depression 4 of the first well 1 was 5 × 10³.

In the case where the number of cells was 5 × 10³, the axons of the neurons cultured could not sufficiently block the flow channel 3. When the culture medium in the second well 2 was sucked, the culture medium in the first well 1 flowed into the second well 2 from the flow channel 3. Thus, a culture medium for co-culture could not be added into the second well 2 separately.

### (Comparative Example 1-2)

The same culture process as in Example 1-1 was performed, except that the number of motor neurons cultured was different from Example 1-1. Specifically, the number of motor neurons cultured in the first depression 4 of the first well 1 was 5 × 10⁴.

In the case where the number of cells was 5 × 10⁴, the nutrient components did not reach the inside of the cell mass, causing the cells inside to die. As a result, a sufficient number of axons could not be cultured. When the culture medium in the second well 2 was sucked, the culture medium in the first well 1 flowed into the second well 2 from the flow channel 3. Thus, a culture medium for co-culture could not be added into the second well 2 separately.

### (Comparative Example 2-1)

The same test and cell culture process as in Example 2-1 were performed, except that the length of the flow channel 3 in the longitudinal direction was 1.5 mm.

Immediately after adding the culture medium for neuron culture and pure water into the respective wells, the culture medium for neuron culture in the first well 1 and the pure water in the second well 2 began to mix through the flow channel 3. Mixing of the culture medium and the pure water was clearly visible after 24 hours.

Thus, the co-culture could not be performed without mixing of the culture medium for neuron culture in the first well 1 and the culture medium for human skeletal muscle cell culture in the second well 2.

### (Comparative Example 2-2)

The same test and cell culture process as in Example 2-1 were performed, except that the depth of the flow channel 3 was 18 µm.

It was confirmed that the culture medium for neuron culture in the first well 1 and the pure water in the second well 2 did not mix through the flow channel 3 even after 24 hours, as in Example 1. However, since the flow channel 3 was excessively narrow, the axons a extending from the motor neurons could not pass through the flow channel 3.

### (Comparative Example 2-3)

Preparation to perform the same test and cell culture process as in Example 2-1 was made, except that the width of the flow channel 3 in the direction orthogonal to the longitudinal direction was 18 µm.

However, a die for a culture vessel for forming a flow channel having the foregoing width could not be produced, and therefore a culture vessel could not be formed.

### (Comparative Example 2-4)

The same test and cell culture process as in Example 2-1 were performed, except that the culture vessel was made of a COP, the width of the flow channel 3 in the direction orthogonal to the longitudinal direction was 80 µm, the depth of the flow channel 3 was 80 µm, and the length of the flow channel 3 in the longitudinal direction was 1.5 mm.

Immediately after adding the culture medium for neuron culture and pure water into the respective wells, the culture medium for neuron culture in the first well 1 and the pure water in the second well 2 began to mix through the flow channel 3. Mixing of the culture medium and the pure water was clearly visible after 24 hours.

Thus, the co-culture could not be performed without mixing of the culture medium for neuron culture in the first well 1 and the culture medium for human skeletal muscle cell culture in the second well 2.

### (Comparative Example 2-5)

The same test and cell culture process as in Example 2-1 were performed, except that culture vessel was made of a COP, the width of the flow channel 3 in the direction orthogonal to the longitudinal direction was 80 µm, the depth of the flow channel 3 was 18 µm, and the length of the flow channel 3 in the longitudinal direction was 1.5 mm.

It was confirmed that the culture medium for neuron culture in the first well 1 and the pure water in the second well 2 did not mix through the flow channel 3 even after 24 hours, as in Example 1. However, since the flow channel 3 was excessively narrow, the axons a extending from the motor neurons could not pass through the flow channel 3.

### (Comparative Example 2-6)

The same test and cell culture process as in Example 2-1 were performed, except that the culture vessel was made of a COP, the width of the flow channel 3 in the direction orthogonal to the longitudinal direction was 150 µm, and the depth of the flow channel 3 was 150 µm.

Immediately after adding the culture medium for neuron culture and pure water into the respective wells, the culture medium for neuron culture in the first well 1 and the pure water in the second well 2 began to mix through the flow channel 3. Mixing of the culture medium and the pure water was clearly visible after 24 hours.

Thus, the co-culture could not be performed without mixing of the culture medium for neuron culture in the first well 1 and the culture medium for human skeletal muscle cell culture in the second well 2.

### INDUSTRIAL APPLICABILITY

With the culture method and the culture vessel according to the present disclosure, in a culture vessel having a plurality of wells, the cell bodies of neurons and the tips of the axons extending from the cell bodies can be cultured under separate suitable culture medium conditions. Moreover, the cell bodies of neurons and the tips of the axons and other cells to be joined to the tips of the axons, such as skeletal muscle cells, can be cultured under separate suitable culture medium conditions. It is thus possible to perform culture using a different culture medium in each well depending on the cells cultured in the well, without making the culture process complex. Hence, neural networks by a plurality of types of cells can be efficiently formed between a plurality of wells.

### REFERENCE SIGNS LIST

- 1: first well
- 2: second well
- 3: flow channel
- 4: first depression
- 5: positioning depression
- 6: positioning projection
- 7: second depression
- B: well body
- D: member forming part of well bottom
- N: neuron
- s: cell body
- a: axon
- C: cell to be co-cultured

## Claims

1. A culture method of co-culturing a plurality of types of cells including neurons in a culture vessel having a plurality of wells, the culture method comprising:
filling a first well and a second well included in the plurality of wells in the culture vessel with a culture medium for the neurons, and seeding the neurons in the first well, the first well and the second well communicating with each other through a flow channel;
culturing axons of the neurons until the axons of the neurons reach the second well through the flow channel and also block the flow channel; and
thereafter removing the culture medium in the second well, filling the second well with a culture medium for co-cultured cells to be co-cultured with the neurons, seeding the co-cultured cells in the second well, and culturing the co-cultured cells together with the axons of the neurons.

2. A culture method of culturing neurons in a culture vessel having a plurality of wells, the culture method comprising:
filling a first well and a second well included in the plurality of wells in the culture vessel with a culture medium for the neurons, and seeding the neurons in the first well, the first well and the second well communicating with each other through a flow channel;
culturing axons of the neurons until the axons reach the second well through the flow channel and also block the flow channel; and
thereafter removing the culture medium in the second well, filling the second well with a culture medium for culturing the axons, and culturing the axons.

3. The culture method according to claim 1 or 2, wherein the first well has a first depression capable of storing a cell mass, at a bottom thereof, and
a cell mass of the neurons is seeded in the first depression.

4. The culture method according to claim 1 or 2, wherein the neurons are central neurons or peripheral neurons.

5. A culture vessel comprising a plurality of wells and configured to culture neurons or co-culture a plurality of types of cells including the neurons,
wherein the plurality of wells include:
a first well having a first depression capable of storing a cell mass, at a bottom thereof; and
a second well communicating with the first well through a flow channel, and
the flow channel has a maximum width of 20 µm to 100 µm in a direction orthogonal to a longitudinal direction thereof, and a depth of 20 µm to 100 µm.

6. The culture vessel according to claim 5, wherein the neurons are peripheral neurons, and
the number of the peripheral neurons seeded in the first depression is 1 × 10⁴ to 4 × 10⁴, and the flow channel has a maximum cross-sectional area of 4 × 10⁻⁶ cm² to 1 × 10⁻⁴ cm².

7. The culture vessel according to claim 5, wherein the neurons are central neurons, and
the number of the central neurons seeded in the first depression is 4 × 10⁴ to 8 × 10⁴, and the flow channel has a maximum cross-sectional area of 4 × 10⁻⁶ cm² to 1 × 10⁻⁴ cm².

8. The culture vessel according to claim 5, wherein the second well has a second depression capable of storing a cell mass, at a bottom thereof.

9. A culture vessel comprising a plurality of wells and configured to culture neurons or co-culture a plurality of types of cells including the neurons,
wherein the plurality of wells include:
a first well having a first depression capable of storing a cell mass, at a bottom thereof; and
a second well communicating with the first well through a flow channel,
one end of the flow channel is connected to the bottom of the first well, and an other end of the flow channel is connected to a bottom of the second well, and
the flow channel has a maximum width of 20 µm to 120 µm in a direction orthogonal to a longitudinal direction thereof, a depth of 20 µm to 120 µm, and a length of 2 mm to 6 mm in the longitudinal direction.

10. The culture vessel according to claim 9, wherein a water contact angle of at least a surface of a part to be in contact with the neurons is 90° to 15°.

11. The culture vessel according to claim 9, wherein at least a surface of a part to be in contact with the neurons is made of an alicyclic structure-containing polymer.

12. A culture method of co-culturing a plurality of types of cells including neurons using the culture vessel according to any of claims 9 to 11, the culture method comprising:
filling the first well in the culture vessel with a culture medium for the neurons, and filling the second well communicating with the first well through the flow channel with a culture medium for co-cultured cells to be co-cultured with the neurons;
seeding the neurons in the first well;
seeding the co-cultured cells in the second well;
culturing axons of the neurons until the axons of the neurons reach the second well through the flow channel; and
thereafter culturing the axons of the neurons together with the co-cultured cells in the second well.

13. A culture method of culturing neurons using the culture vessel according to any of claims 9 to 11, the culture method comprising:
filling the first well in the culture vessel with a culture medium for the neurons, and filling the second well communicating with the first well through the flow channel with a culture medium for culturing axons;
seeding the neurons in the first well;
culturing axons of the neurons until the axons reach the second well through the flow channel; and
thereafter culturing the axons in the second well.
